(19) Europäisches Patentamt
European Patent Office
Office européen des brevets

(11) Publication number: **0 557 462 B1**

# (12) EUROPEAN PATENT SPECIFICATION

(45) Date of publication of patent specification: **09.08.95**

(51) Int. Cl.[6]: **C07C 231/12**

(21) Application number: **92902536.9**

(22) Date of filing: **07.11.91**

(86) International application number:
**PCT/SE91/00752**

(87) International publication number:
**WO 92/08690 (29.05.92 92/12)**

## (54) METHOD FOR PRODUCING ETHANOLAMIDE ALKOXYLATE.

(30) Priority: **12.11.90 SE 9003596**

(43) Date of publication of application:
**01.09.93 Bulletin 93/35**

(45) Publication of the grant of the patent:
**09.08.95 Bulletin 95/32**

(84) Designated Contracting States:
**BE DE FR GB IT NL**

(56) References cited:
**DE-A- 2 365 751**
**US-A- 4 670 591**

**CHEMICAL ABSTRACTS, Volume 92, No. 1, 7 January 1980, (Columbus, Ohio, US), seepage 573, Abstract 6095f, & PO, A, 102560 (Fatty acid ethanolamides) 1979.**

(73) Proprietor: **BEROL NOBEL AB**
**S-444 85 Stenungsund (SE)**

(72) Inventor: **SANDBERG, Elina**
**Lunden 13961**
**S-444 95 Ödsm I (SE)**

(74) Representative: **Andersson, Rolf**
**Berol Nobel AB**
**Patentavdelningen**
**S-444 85 Stenungsund (SE)**

EP 0 557 462 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for producing high yields of ethanolamide alkoxylate with low contents of undesired by-products.

It is a well-known fact, disclosed e.g. in an article by H. Grossmann in Fette-Seifen-Anstrichmittel, No. 1, 74 (1972), pp 58-63, that non-ionic surface-active compounds can be produced by ethoxylation of fatty acid monoethanolamides in the presence of an alkaline catalyst at a temperature of 150-180 °C. NaOH, KOH, $NaOCH_3$ and $KOCH_3$ have been used as catalyst. If a fatty acid monoethanolamide is reacted with 5 moles of ethylene oxide per mole of amide, this results, according to the article, in a reaction mixture of the following approximate composition:

| | | | |
|---|---|---|---|
| I | Fatty acid amide ethoxylate | $RCONH(CH_2CH_2O)_x H$ | 80% |
| II | Ester amine ethoxylate | 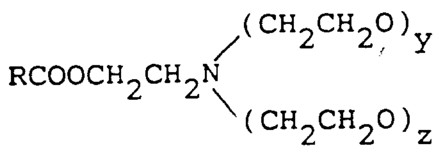 | 6% |
| III | Nitrilotripolyglycol ether | 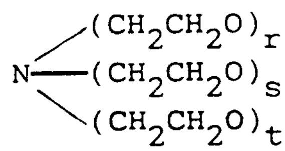 | 8% |
| IV | Polyglycol ether | $HO(CH_2CH_2O)_u H$ | 2% |
| V | Fatty acid polyglycol ester | $RCOO(CH_2CH_2O)_v H$ | 4% |

Being cationic, especially the by-products II and III are undesirable. Despite the fact that ethoxylated fatty acid monoethanolamides have been commercially available for more than 30 years, they still constitute but a small part of the total amount of non-ionic surface-active agents. This should mainly be attributed to the fact that it has only been possible to produce impure products as above, unless one resorted to costly processing methods. Thus, there is a manifest need of carboxylic acid ethanolamide ethoxylate in high yields and with low contents of by-products.

The present invention relates to a new method for alkoxylating monoethanolamides, thereby obtaining compounds of type I in a yield of at least 95%, at the same time as the undesired compounds of types II and III are present in contents less than 1% by weight. According to the invention, ethanolamide alkoxylate of the general formula

$R^I CONHC_2H_4O(A)_xH$

wherein $R^I$ is a hydrocarbon group having 1-29 carbon atoms, A is an alkyleneoxy group derived from an alkylene oxide having 2-4 carbon atoms, and x is 2-30, is produced by reacting the corresponding carboxylic acid monoethanolamide with an alkylene oxide having 2-4 carbon atoms in the presence of a tertiary amine lacking protons that react with alkylene oxide, or an alkylene-oxide-quaternised derivative of the tertiary amine at a temperature below 120 °C, preferably 50-100 °C. In the reaction conditions, the tertiary amine probably forms a zwitter compound with alkylene oxide in accordance with the reaction formula

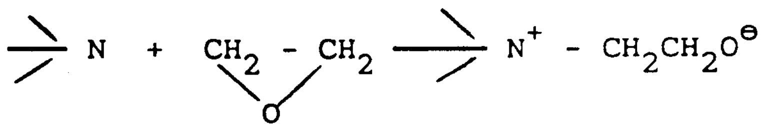

whereupon the zwitterion compound reacts with the ethanolamide. The zwitterion compound is more basic than the amine from which it originates. Extensive tests have shown that, at temperatures exceeding 120°, other reaction mechanisms set in, which suppress the formation of the desired compounds. The suitable reaction temperature according to the invention ranges from room temperature to 120°C, preferably 50-100°C, most preferred 70-80°C.

As mentioned earlier, the tertiary amine must not contain any protons that react with alkylene oxide. The nitrogen groups usually have substituents that contain hydrocarbon groups, such as acyclic hydrocarbon groups, cycloaliphatic or aromatic groups, or form a cyclic group with a divalent hydrocarbon group. The substituents may also contain other atoms which do not react with alkylene oxide, such as oxygen atoms in ether groups. Suitable tertiary amine compounds include trimethylamine, triethylamine, tributylamine, dimethyloctylamine, tetramethylethylenediamine, dimethyl coconut amine, tristearyl amine, and cyclic amines, such as dimethyl piperazine and diazabicyclooctane (Dabco). The preferred amine catalysts contain at least one substituent that consists of an alkyl group having 1-4 carbon atoms.

The amount of catalyst suitably is less than 15 mole% of the amount of ethanolamide. Higher contents of the catalyst do not increase the reaction rate. For practical reasons, the catalyst content should preferably be 5-8 mole%.

The carboxylic acid ethanolamide can actually be based on all types of monocarboxylic acids. Usually, the carboxylic acids contain 2-30 carbon atoms, preferably 8-20 carbon atoms. They may further be either synthetically produced or naturally derived.

In the alkoxylation, ethylene oxide, propylene oxide or butylene oxide can be added in one or more steps. If desired, several different alkylene oxides can be added in the same molecule, e.g. by random addition or stepwise addition of blocks of specific alkylene oxides, or by using both principles at the same time.

The ethanolamide alkoxylates can be used in a number of different detergent compositions, e.g. such compositions as are used for cleaning textiles and hard surfaces.

The present invention will now be further illustrated with the aid of the following Examples.

Example 1

Rape fatty acid monoethanolamide in an amount of 450 g (1.36 mole) was melted and batched in an ethoxylation reactor. Then, the reactor was treated with nitrogen gas, and 3.5 g of trimethylamine was batched as catalyst. The reactor temperature was raised to 75°C, and 244 g (5.44 mole) of ethylene oxide was fed to the reactor under intense cooling. The temperature was maintained at 75-80°C. After 20 min. when all the ethoxylene oxide had been batched, the temperature was raised to 80°C for 20 min. The resulting reaction mixture was vacuum-treated in the reactor, so that essentially all the catalyst was stripped, whereupon ethanol/water was added to strip the remaining amine and formed dioxane. The resulting reaction product was a bright yellow liquid having a cloud point of 81°C in an aqueous solution containing 25% by weight of butyl diethylene glycol. Potentiometric titration with 0.1 M HCl showed that the undesired products II and III amounted to about 0.4% by weight.

Example 2

Here, 560 g of linseed oil fatty acid monoethanolamide was batched in an ethoxylation reactor together with 13.18 g of the catalyst triethylamine after careful nitrogen-gas treatment. The reactor was heated to 80°C, and 173 g of ethylene oxide was supplied. The reactor temperature was maintained at 80°C. After 5.5 h, the reactor was evacuated and the triethylamine removed under vacuum. Potentiometric titration showed that the content of the undesired compounds II and III did not exceed about 0.5% by weight.

Example 3

Coconut fatty acid monoethanolamide in an amount of 890 g (3.34 mole) was hatched in a reactor together with 78.7 g of dimethyloctylamine after careful nitrogen-gas treatment of the reactor. After a temperature increase to 80 °C, 885 g of ethylene oxide was added for 1 h under intense cooling. After another 15 min. at 80 °C, the reaction was interrupted, and the reaction mixture was treated with $KH_2PO_4$ and filtered in order to remove dimethyloctylamine. After filtering, potentiometric titration with 0.1 M HCl was unable to indicate the presence of any tertiary amine. The resulting reaction product had a cloud point of 58 °C in an aqueous solution containing 10% by weight of NaCl.

Example 4

Tall oil fatty acid monoethanolamide in an amount of 640 g (1.9 mole) and 5.7 g of dimethyl piperazine were batched in a reactor which previously had been treated with nitrogen gas. Then, the reactor was heated to 70 °C, and 251 g (5.7 mole) of ethylene oxide was batched at 70 °C. The resulting reaction mixture was treated with acid ion exchanger in order to remove the catalyst. The resulting end product had extremely low contents of the tertiary nitrogen compound and further had a cloud point of 5 °C in water and 96 °C in water containing 25% by weight of butyl diethylene glycol.

Example 5

In the manner described above, 365 g (8,28 mole) of ethylene oxide was added to tall oil fatty acid monoethanolamide (1.9 mole) at 70 °C. Tetramethylethylenediamine in an amount of 0.02 mole was used as catalyst, and was removed after the completed reaction by filtering with $KH_2PO_4$. The resulting end product had a cloud point of 27 °C in water containing 10% NaCl. Gas chromatography showed that compounds of type I were obtained in a yield of at least 98%.

**Claims**

1. Method for producing ethanolamide alkoxylate of the general formula

$R^ICONHC_2H_4O(A)_xH$

wherein $R^I$ is a hydrocarbon group having 1-29 carbon atoms, A is an alkyleneoxy group derived from an alkylene oxide having 2-4 carbon atoms, and x is 2-30, 2-30 moles of alkylene oxide having 2-4 carbon atoms being added to a compound of the formula

$R^ICONHC_2H_4OH$

wherein $R^I$ and n have the meanings stated above,
**characterised** in that the alkylene oxide is added in the presence of a tertiary amine lacking protons that react with alkylene oxide, or an alkylene-oxide-quaternised derivative of the tertiary amine as a catalyst at a temperature ranging from room temperature to 120 °C.

2. The method of claim 1, **characterised** in that the addition is carried out at a temperature of 50-100 °C.

3. The method of claim 1 or 2, **characterised** in that the tertiary amine is a trialkyl amine, at least one of the alkyl groups having 1-4 carbon atoms.

4. The method of claim 1 or 2, **characterised** in that the catalyst is a tetraalkylene diamine.

5. The method of claim 1 or 2, **characterised** in that the catalyst is a cyclic tertiary amine compound.

6. The method of any one of claims 1-6, **characterised** in that the tertiary amine or the alkylene-oxide-quaternised derivative thereof is present in an amount less than 15 mole%, preferably 5-8 mole%, as based on the amount of ethanolamide.

## Patentansprüche

1. Verfahren zur Herstellung von Ethanolamidalkoxylaten der allgemeinen Formel

$R^ICONHC_2H_4O(A)_xH$

worin $R^I$ eine Kohlenwasserstoffgruppe mit 1-29 Kohlenstoffatomen ist, A eine Alkylenoxygruppe ist, die sich von einem Alkylenoxid mit 2-4 Kohlenstoffatomen ableiten, und x 2-30 ist, wobei 2-30 Mol des Alkylenoxids mit 2-4 Kohlenstoffatomen zu einer Verbindung der Formel

$R^ICONHC_2H_4OH$

gegeben werden, worin $R^I$ und n die oben angegebene Bedeutung haben,
dadurch gekennzeichnet, daß das Alkylenoxid in Gegenwart eines tertiären Amins, das keine mit dem Alkylenoxid reagierenden Protonen aufweist, oder einem Alkylenoxid-quaternisierten Derivat des tertiären Amins als Katalysator bei einer Temperatur im Bereich von Raumtemperatur bis 120 °C zugegeben wird.

2. Verfahren nach Anspruch 1, dadurch gekennzeichnet, daß die Zugabe bei einer Temperatur von 50-100 °C durchgeführt wird.

3. Verfahren nach Anspruch 1 oder 2, dadurch gekennzeichnet, daß das tertiäre Amin ein Trialkylamin ist, bei dem mindestens eine der Alkylgruppen 1-4 Kohlenstoffatome aufweist.

4. Verfahren nach Anspruch 1 oder 2, dadurch gekennzeichnet, daß der Katalysator ein Tetraalkylendiamin ist.

5. Verfahren nach Anspruch 1 oder 2, dadurch gekennzeichnet, daß der Katalysator eine cyclische tertiäre Aminverbindung ist.

6. Verfahren nach irgendeinem der Ansprüche 1-6, dadurch gekennzeichnet, daß das tertiäre Amin oder das Alkylenoxid-quaternisierte Derivat davon in einer Menge von weniger als 15 Mol%, vorzugsweise 5-8 Mol%, bezogen auf die Menge an Ethanolamid, vorhanden ist.

## Revendications

1. Procédé de production d'alcoxylate d'éthanolamide de formule générale

$R^ICONHC_2H_4O(A)_xH$

dans laquelle $R^I$ représente un groupe hydrocarboné comportant 1-29 atomes de carbone, A représente un groupe alkylèneoxy dérivé d'un oxyde d'alkylène comportant 2-4 atomes de carbone et x a une valeur de 2 à 30, qui consiste à ajouter 2-30 moles d'oxyde d'alkylène comportant 2-4 atomes de carbone à un composé de formule

$R^ICONHC_2H_4OH$

dans laquelle $R^I$ et n ont les significations indiquées ci-dessus,
caractérisé en ce que l'oxyde d'alkylène est ajouté en présence, comme catalyseur, d'une amine tertiaire dépourvue de protons réagissant avec l'oxyde d'alkylène, ou d'un dérivé quaternisé par l'oxyde d'alkylène de l'amine tertiaire, à une température allant de la température ambiante à 120 °C.

2. Procédé selon la revendication 1, caractérisé en ce que l'addition est réalisée à une température de 50-100 °C.

3. Procédé selon la revendication 1 ou 2, caractérisé en ce que l'amine tertiaire est une trialkylamine, dont au moins un des groupes alkyle comporte 1-4 atomes de carbone.

4. Procédé selon la revendication 1 ou 2, caractérisé en ce que le catalyseur est une tétraalkylènediamine.

5. Procédé selon la revendication 1 ou 2, caractérisé en ce que le catalyseur est un composé amine tertiaire cyclique.

6. Procédé selon l'une quelconque des revendications 1-5, caractérisé en ce que l'amine tertiaire, ou son dérivé quaternisé par l'oxyde d'alkylène, est présente à raison de moins de 15% en moles, de préférence de 5-8% en moles, sur la base de la quantité d'éthanolamide.